# EUROPÄISCHE PATENTSCHRIFT

(11) **EP 1 384 680 B1**
(45) Veröffentlichungstag und Bekanntmachung des Hinweises auf die Patenterteilung: **21.02.2007**
(21) Anmeldenummer: 03015054.4
(22) Anmeldetag: 03.07.2003
(51) Int. Cl.: B65D 75/38, B65D 85/808

(54) **Kräuterpackung zur Herstellung eines Heil- und Pflegebades mit rückfettenden Substanzen**
Herb package with emollients for medicinal baths
Emballage d'herbes avec des émollients pour bains médicinaux

(30) Priorität: 24.07.2002 DE 10233478
(43) Veröffentlichungstag der Anmeldung: 28.01.2004
(73) Patentinhaber: August Töpfer & Co. (GmbH & Co.), 20539 Hamburg (DE)
(72) Erfinder: Ernst, Rudolf, 22607 Hamburg (DE)
(74) Vertreter: Fleck, Thomas

(56) Entgegenhaltungen:
- EP-A- 0 750 905
- EP-A- 1 157 940
- FR-A- 1 263 098

## Beschreibung

Die Erfindung betrifft eine Kräuterpackung zur Herstellung eines Heil- und Pflegebades mit rückfettenden Substanzen nach dem Oberbegriff des Anspruchs 1.

Eine derartige Kräuterpackung ist durch die DE 100 25 677 C1 vorbekannt. Diese Packungen bestehen aus getrockneten Kräutern, welche mit oder ohne weitere Zusatzstoffe in Filterbeuteln aus Papier oder Polyolefingewebe oder Vlies abgefüllt sind. Der Anwender soll diese Beutel in das Badewasser geben, damit die wasserlöslichen Inhaltsstoffe ausgespült und die Kräuter selbst zurückgehalten werden. Weiterhin ist bekannt, daß reine Kräuterpackungen eine unbefriedigende Wirkung haben. Nachteilig hat sich bemerkbar gemacht, daß die Erwartungen des Anwenders in Bezug auf die Wassereinfärbung, den Geruch bzw. das Aroma und die rückfettende Wirkung auf der Haut von reinen Kräuterzubereitungen nicht erfüllt werden können. Badewasser, das -mit reinen Kräuterpackungen ohne Zusätze zubereitet wird, entfettet vielmehr die Haut.

Deshalb hatte der eingangs genannte Stand der Technik auch schon Farbstoffe zur Intensivierung der Badewasserdurchfärbung, ätherische Öle oder andere Duftstoffe zur Verbesserung des Geruchserlebnisses zugesetzt, wodurch das Badewasser in befriedigender Weise gefärbt und duftend verändert werden konnte. Auch wurde schon als rückfettende Substanz Molkepulver als Zusatz zu den getrockneten Kräutern durch das eingangs genannte Patent vorgeschlagen. Auf diese Weise kann man tatsächlich eine rückfettende Wirkung erzielen. Nachteilig hat sich jedoch bemerkbar gemacht, daß der anspruchsvolle Verbraucher eine stärkere Wirkung erwartet, als auf die vorbekannte Weise erzielt werden konnte. Bisher gibt es keine Lösung für eine Kräuterpackung, die dem Badewasser eine perfekte rückfettende Wirkung verleiht. Letzteres ist jedoch äußerst wichtig, weil nur durch rückfettende Substanzen die Wirkung eintritt, die der anspruchsvolle Verbraucher von einem hochwertigen Badeprodukt erwartet.

Im Stand der Technik ist ferner die Verpackung von - vorwiegend flüssigen - Badezusätzen in wasserlösliche Packungsmittel bekannt. So sind z.B. kugelförmige Produkte in Handel, die diverse Öle, nicht jedoch getrocknete Kräuter enthalten.

Das der Erfindung zugrundeliegende Problem liegt darin, daß es nicht möglich ist, eine ausreichend große Menge an rückfettenden Substanzen mit den entsprechenden Emulgatoren der Kräutermischung zuzumischen, da solche Zumischungen in der gewünschten Größenordnung, die mindestens bei 4g, insbesondere jedoch zwischen 22 und 25g liegen sollte, das System instabil machen. Die Öle fangen nämlich an, zu diffundieren und fetten das Filterpapier oder das Polyolefingewebe durch, so daß die Packungen unansehnlich und schmierig werden, was zu verhindern ist.

Die skizzierten Probleme werden erfindungsgemäß dadurch gelöst, daß die rückfettenden Substanzen in einer Menge > 4g in einer wasserlöslichen Folienpackung aus Polyvinylalkohol (PVAL) abgefüllt sind, die neben den getrockneten Kräutern etc. im Vlies- oder Gewebebeutel angeordnet ist. Insbesondere ist auch daran gedacht, größere Mengen an rückfettenden Substanzen einzusetzen, vorteilhafterweise 5 bis 10g und darüber hinaus.

Die erfindungsgemäße Lösung besteht also darin, die rückfettenden Substanzen, zu denen auch entsprechende Emulgatoren gehören, zunächst in wasserlösliche Folie aus PVAL zu verpacken und diese Packung zusammen mit den Kräutern und ggf. weiteren Zusatzstoffen in dem PP-Gewebebeutel zu verpacken. Dieser Gewebebeutel wird dann bekanntermaßen diffusions- und aromadicht eingesiegelt. Selbstverständlich können auch mehrere rückfettende Substanzen enthaltende PVAL-Folienpacks in dem PP-Gewebebeutel enthalten sein.

Die erfindungsgemäße Kräuterpackung kann leicht und schnell durch den Verbraucher gehandhabt werden: dieser öffnet die aromadichte Umverpackung und legt den Gewebebeutel ins Badewasser. Die wasserlöslichen Farb- und Duftstoffe gehen sofort in Lösung. Das Badewasser beginnt, die löslichen Stoffe aus den Kräutern herauszulösen. Die PVAL Folienpackung oder -Beutel wird vom Badewasser aufgelöst und gibt die rückfettenden Substanzen frei. Nach dem Ende des Bades wird der Gewebebeutel vom Verbraucher entsorgt. Die PVAL Folienpackung hat sich rückstandslos im Badewasser aufgelöst, so daß sich der Verbraucher in tatsächlicher Hinsicht nicht um mehrere, sondern nur um einen Beutel kümmern muß.

Weitere Vorteile und Merkmale gehen aus den Unteransprüchen hervor, die auch gemeinsam mit dem Hauptanspruch von erfinderischer Bedeutung sein können.

Im folgenden wird ein bevorzugtes Ausführungsbeispiel zum besseren Verständnis der Erfindung anhand der Zeichnungen näher erläutert. Es zeigt:
- Fig. 1: eine schematische und vergrößerte Querschnittsansicht der erfindungsgemäßen Kräuterpackung; und
- Fig. 2: eine schematische Draufsicht auf die in Fig. 1 gezeigte erfindungsgemäße Kräuterpackung.

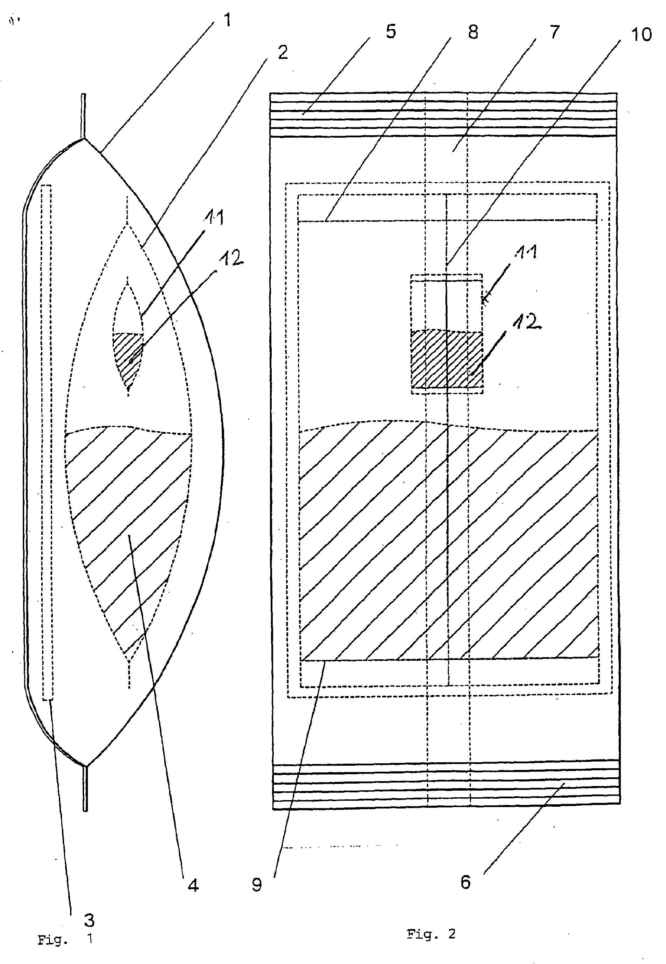

Die in Fig. 1 gezeigte Kräuterpackung weist im Querschnitt eine in etwa ovale Form auf, während sie in der Draufsicht der Fig. 2 eine in etwa rechteckige Form besitzt. Sowohl die Form, als auch die Größe der erfindungsgemäßen Kräuterpakkung und ihrer Einzelbestandteile wie Gewebebeutel 2, PVA Beutel 11 und Folienumbeutel 1 ist nicht irgendwie kritisch, sondern kann vom Fachmann im geeigneten Rahmen beliebig verändert werden. Wichtig ist hingegen die Anordnung.des PVAL Beutels 11 mit dem bzw. den Ölen 12 und Emulgator(en) innerhalb des Gewebebeutels 2, Die Beutelbreite des PVA Beutels 11 kann beispielsweise 50 mm betragen, bei einer Beutelhöhe von etwa 55-60 mm bei einer Dicke von circa 50-80 µm des Beutels 11. Andere Werte sind jedoch für den Fachmann ebenfalls denkbar.

Der Folienumbeutel 1 weist eine geschweißte oder gesiegelte Bodennaht 6 bzw. Rückennaht 7 sowie eine Kopfnaht 5 auf. Er ist diffusionsdicht und läßt somit eine einwandfreie Lagerung und Transport von den Produkten und Stoffen zu, die in seinem Inneren aufgenommen sind. In dem Folienumbeutel 1 befindet sich zudem eine Pappaussteifung 3, die für den Beuteltransport in einer horizontalen Schlauchbeutelmaschine erforderlich ist. Darüber hinaus ermöglicht sie dem gesamten Produkt eine stabilere Form und trägt zu einer einfachen und leichten Handhabung bei. Die eigentlichen Wirkstoffe sind von einem PP- oder PE-Vlies- oder Gewebebeutel 2 aufgenommen, und zwar neben den Kräutern ebenfalls Badesalz, ggf. Öle und Farbstoffe, die insgesamt mit 4 bezeichnet sind. Die Mengen der einzelnen Bestandteile und sie selbst sind nicht kritisch, sondern können in geeignetem Rahmen verändert werden, um gewisse Wirkstoffeigenschaften hervorzuheben. Ebenso wie der Folienumbeutel 1 enthält der in seinem Inneren angeordnete Vlies- oder Gewebebeutel 2 eine Kopfnaht 8, Bodennaht 9 und Rückennaht 10. Sämtliche Nähte sind herstellungstechnisch bedingt und nicht als kritisch anzusehen.

Zur Anwendung der erfindungsgemäßen Kräuterpackung reißt der Benutzer den Folienumbeutel 1 auf und entfernt die Pappaussteifung 3. Er bringt dann den PP- oder PE-Vlies oder Gewebebeutel 2 mit seinen sämtlichen Wirkstoffen 4 und dem wasserlöslichen PVAL-Beutel 11 mit dem bzw. den Ölen 12 und Emulgator(en) in das nicht gezeigte Badewasser, in dem sich die wasserlöslichen Teile auflösen und mit den rückfettenden Ölen 12 eine Emulsion bildet. Gleichzeitig werden die Wirkstoffe der Kräuter langsam ausgespült, während die ausgelaugten Kräuter im Gewebebeutel 2 zurückgehalten werden.

Die rückfettenden Substanzen der erfindungsgemäßen Kräuterpackungen basieren auf der nachstehenden Basisrezeptur, deren Werte in Gew.-% angegeben sind:

| | |
|---|---|
| Ölkomponente (pflanzliche Öle wie Mandelöl, Sojaöl etc.) | 50-93% |
| Emulgatoren, öllöslich | 4-20% |
| Emulgatoren, wasserlöslich; Spreitungsmittel | 2-20% |
| Stabilisator (z.B. Antioxidantien wie Tocopherol) | 1-5% |

Als Ölkomponente können neben pflanzlichen Ölen wie Mandelöl, Weizenkeimöl, Nachtkerzenöl und Jojobaöl auch noch Paraffin und künstliche Ölkomponenten, wie Isopropylmyristat oder -palmitat, Dicaprylether, Caprylic/Caprictriglyceride, Isohexadecan oder Cyclomethicone oder Dimethicone, zwei Siliconöle eingesetzt werden.

Der Anteil an den obigen Ölen in einem erfindungsgemäßen Ölbad wird eher größer als 50%, wenn das Produkt wasserfrei formuliert ist. Die obere Grenze für den Ölgehalt kann bei 90% bis 93% liegen.

Als lipophile Emulgatoren können Sorbitantrioleate, Glycerinester, wie Polyglyceryl-3 diisostearate, Zuckerester oder auch PEG-haltige Emulgatoren wie Steareth-2 oder PEG-2 Stearate eingesetzt werden. Das erfindungsgemäße Ölbad sollte am besten eine Kombination von lipophilen und hydrophilen Emulgatoren enthalten.

Als hydrophile Emulgatoren können ethoxilierte Sorbitanester, Glycerinester, wie Polyglyceryl-4caprate, Zuckerester oder auch PEG-haltige Emulgatoren wie Steareth-20 oder PEG-20 Stearate eingesetzt werden.

Desweiteren kann das erfindungsgemäße Ölbad Überfettungsmittel wie Cocosfettsäurediethanolamid oder ein anderes Säureamid enthalten. Häufig verwendete Überfettungsmittel sind auch PEG-7 Glyceryl Cocoate oder ethoxilierte Wollfette.

Einfache Ölbäder können auch PEG-40 hydroginated Castor Oil enthalten.

Als Pflegekomponenten sollten vorteilhafterweise Lecithine und Linolsäure und Linolensäure vorhanden sein.

Als Anitoxidans können Tocopherol, Tocopherolacetat, Ascorbylpalmitat, Butylhydroxyanisol und/oder Butylhydroxytoluol eingesetzt werden.

Eine vorteilhafte Rezeptur ist nachstehend mit Stoffen und Anteil in % angegeben:

| | |
|---|---|
| Mandelöl | 87,50% |
| Sorbitantrioleate | 4,00% |
| Polyglyceryl-3 diisostearate | 2,00% |
| Cocosfettsäure- diethanolamid | 3,00% |
| nat. Vitamin E | 0,50% |
| Polyglyceryl-4 isostearate | 3,00% |
| Summe: | 100,00% |

## Patentansprüche

1. Kräuterpackung zur Herstellung eines Heil- und Pflegebades mit rückfettenden Substanzen, umfassend einen wasserbeständigen Gewebe- oder Vliesbeutel (2) aus PE oder PP, in den neben getrockneten Kräutern Farbstoff(e) und/oder Badesalz und/oder weitere Zusatzstoffe aufgenommen und eingeschweißt sind, und der so wasserdurchlässig ist, daß die wasserlöslichen Wirkstoffe der getrockneten Kräuter durch die Beutelwand hindurch an das Badewasser abgegeben und die Kräuter zurückgehalten werden, und der von einem diffusionsdichten Folien-Umbeutel (1) umgeben ist, welcher zum Gebrauch entfernbar ist, **dadurch gekennzeichnet, daß** die rückfettenden Substanzen in einer Menge > 4g in einer wasserlöslichen Folienpackung (11) aus Polyvinylalkohol abgefüllt sind, die neben den getrockneten Kräutern etc. im Gewebe- oder Vliesbeutel angeordnet ist.

2. Kräuterpackung nach Anspruch 1, **dadurch gekennzeichnet, daß** die rückfettenden Substanzen pflanzliche Öle (12), Emulgatoren und/oder weitere Zusatzstoffe in fester oder flüssiger Form mit einem Wassergehalt < 5 Gew.-% umfassen.

3. Kräuterpackung nach Anspruch 1 und/oder 2, **dadurch gekennzeichnet, daß** die rückfettenden Substanzen Rizinusöl und/oder Mandelöl oder andere pflanzliche Öle (12) in einer Menge von 5 bis 25g umfassen, insbesondere 22 bis 25g.

4. Kräuterpackung nach Anspruch 1 bis 3, **dadurch gekennzeichnet, daß** der diffusionsdichte Folien-Umbeutel (1) aromadicht ist und aus Kunststoff-Verbundfolien, Monofilen aus Polyolefinen, Papierverbunden und/oder Aluminiumverbunden besteht.

5. Verfahren zum Herstellen der Kräuterpackung nach einem oder mehreren der Ansprüche 1 bis 4, **dadurch gekennzeichnet, daß** rückfettende Substanzen in einer Menge > 4g in einer wasserlöslichen Folienpackung (11) aus PVAL als Schlauchbeutel, Siegelrandbeutel oder in Tiefziehpackungen abgefüllt und zusammen mit einer kräuterhaltigen Mischung in einen auf einer Schlauchbeutelmaschine hergestellten, wasserbeständigen PP- oder PE-Gewebe- oder Vliesbeutel (2) eingeschweißt werden, der wiederum seinerseits anschließend zur Konservierung der flüchtigen und ggf. flüssigen Inhaltsstoffe der Mischung auf einer horizontalen Schlauchbeutelmaschine in einen leicht entfernbaren Umbeutel aus diffusionsdichter Folie (1) eingeschweißt wird.

## Claims

1. Herb pack for making a medicinal bath with emollients, comprising a water-resistant, PE or PP woven or nonwoven bag (2), in which are received and sealed dye(s) and/or bath salt and/or further additives in addition to the dried herbs and which is sufficiently water-permeable for the water-soluble agents of the dried herbs to be delivered through the bag wall to the bath water, whilst the herbs are retained, and which is surrounded by a diffusion-tight outer film bag (1), which is removable when use takes place, **characterized in that** the emollients are filled in a quantity of > 4 g into a water-soluble, polyvinyl alcohol film pack (11), placed together with the dried herbs, etc. in the woven or nonwoven bag.

2. Herb pack according to claim 1, **characterized in that** the emollients comprise vegetable oils (12), emulsifiers and/or further additives in solid or liquid form with a water content < 5 wt.%.

3. Herb pack according to claim 1 and/or 2, **characterized in that** the emollients comprise castor oil and/or almond oil or other vegetable oils (12) in a quantity of 5 to 25 g, particularly 22 to 25 g.

4. Herb pack according to claims 1 to 3, **characterized in that** the diffusion-tight outer film bag (1) is aroma-tight and comprises plastic laminated films, polyolefin monofilaments, paper composites and/or aluminium composites.

5. Method for making for herb pack according to one or more of the claims 1 to 4, **characterized in that** the emollients are filled in a quantity of > 4 g into a water-soluble PVAL film pack (11) as a flow pack, sealed edge pack or in deep-drawn packs and sealed with a herb-containing mixture into a water-resistant PP or PE woven or nonwoven bag (2) made on a flow pack machine, which is in turn sealed into an easily removable outer bag made from diffusion-tight film (1) on a horizontal flow pack machine for preserving the volatile and optionally liquid constituents of the mixture.

## Revendications

1. Emballage d'herbes conditionné pour la préparation d'un bain médicinal et curatif contenant des substances émollientes, comprenant une poche (2) en tissu ou en non-tissé résistant à l'eau, en PE ou PP, dans laquelle sont reçus et enfermés par soudage, à côté d'herbes séchées, un ou plusieurs colorants et/ou un sel de bain et/ou d'autres additifs, et dont la perméabilité à l'eau est telle que les substances actives hydrosolubles des herbes séchées soient cédées à l'eau du bain à travers la paroi de la poche et que les herbes soient retenues, et qui est entourée d'une enveloppe (1) en feuille étanche à la diffusion qui doit être retirée au moment de l'utilisation, **caractérisé en ce que** les substances émollientes sont chargées en une quantité supérieure à 4 g dans une feuille d'emballage (11) soluble dans l'eau en polymère d'alcool vinylique, qui est disposée en plus des herbes séchées, etc., dans l'enveloppe en tissu ou en non-tissé.

2. Emballage d'herbes suivant la revendication 1, **caractérisé en ce que** les substances émollientes comprennent des huiles végétales (12), des émulsifiants et/ou d'autres additifs sous forme solide ou liquide, avec une teneur en eau inférieure à 5 % en poids.

3. Emballage d'herbes suivant la revendication 1 et/ou 2, **caractérisé en ce que** les substances émollientes comprennent de l'huile de ricin et/ou de l'huile d'amandes et/ou d'autres huiles végétales (12) en une quantité de 5 à 25 g, en particulier de 22 à 25 g.

4. Emballage d'herbes suivant la revendication 1 à 3, **caractérisé en ce que** l'enveloppe (1) en feuille étanche à la diffusion est étanche aux arômes et constituée de feuilles composites en matière plastique, de monofils en polyoléfines, de composites de papier et/ou de composites d'aluminium.

5. Procédé de fabrication de l'emballage d'herbes suivant une ou plusieurs des revendications 1 à 4, **caractérisé en ce que** des substances émollientes sont chargées en une quantité supérieure à 4 g dans une feuille d'emballage (11) soluble dans l'eau en PVAL réalisée comme poche tubulaire, poche à bords soudés ou dans des emballages emboutis et sont enfermées par soudage conjointement avec un mélange contenant des herbes dans une poche (2) en tissu ou en non-tissé de PP ou de PE résistant à l'eau, réalisée sur une machine de confection tubulaire, qui est ensuite enfermée à son tour par soudage dans une enveloppe facile à retirer, formée d'une feuille (1) étanche à la diffusion, en vue de la conservation des constituants volatils et le cas échéant liquides du mélange, sur une machine horizontale de confection de poches tubulaires.
